# EUROPEAN PATENT APPLICATION

(11) **EP 1 424 052 A1**
(43) Date of publication of application: **02.06.2004**
(21) Application number: 03026925.2
(22) Date of filing: 25.11.2003
(51) Int. Cl.: A61F 13/15

(54) **Absorbent article and method for producing the same**

(30) Priority: 27.11.2002 JP 2002344314; 27.12.2002 JP 2002379009
(71) Applicant: Zuiko Corporation, Osaka 566-0045 (JP)
(72) Inventor: Ichiura, Yuzo, Osaka 567-0845 (JP); Nakakado, Masaki, Osaka 573-0065 (JP); Makimura, Kazutoshi, Osaka 573-0066 (JP); Kurata, Syuhei, Uji Kyoto 6111-0002 (JP)
(74) Representative: Schwabe - Sandmair - Marx

(57) **Abstract**

An absorbent article (1) includes a cuff (30) and a liquid-holding absorbent (5). The cuff (30) is provided on a surface of a sheet (3) covering the liquid-holding absorbent (5). The cuff (30) is obtained by folding the sheet, which faces a surface of a body of a wearer, and folding back the sheet so as to form a two-folded shape. An adhesion section (6a, 6b, 37) for keeping the two-folded shape of the cuff (30) is provided at least near a base portion (35) of the cuff (30).

## Description

### BACKGROUND OF THE INVENTION

### FIELD OF THE INVENTION:

The present invention relates to an absorbent article.

### DESCRIPTION OF THE RELATED ART:

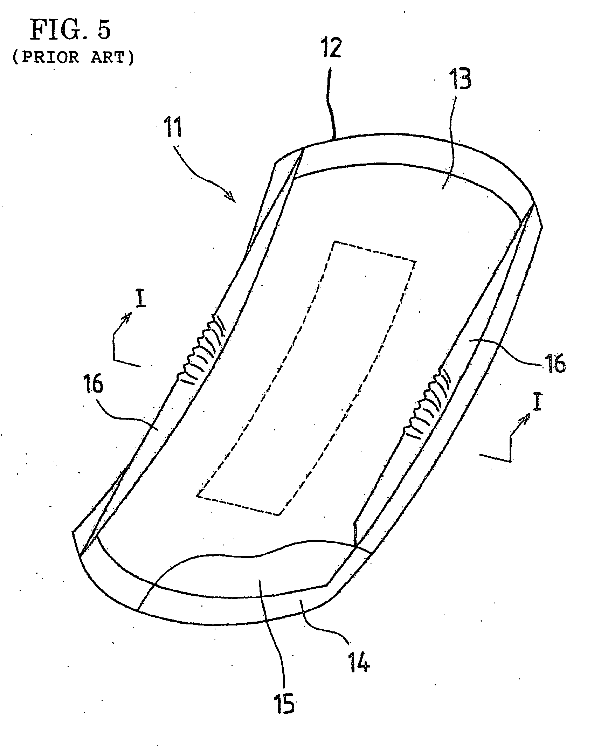

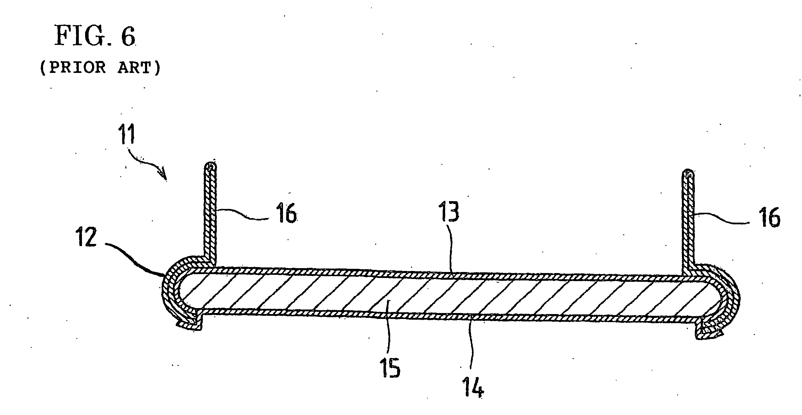

Typically, as illustrated in prior art FIG. 5 and FIG. 6, an absorbent article (e.g., a sanitary napkin) 11 at least includes an elongate main part 12, including a liquid-permeable surface sheet 13, a liquid-impermeable anti-leak sheet 14, and a liquid-holding absorbent 15 provided between the surface sheet 13 and the anti-leak sheet 14. The absorbent article 11 further includes a standing portion 16 made of a liquid-impermeable material (e.g., water-repelling non-woven fabric) along each side edge of the main part 12 in order to ensure that excrement such as menstrual blood is absorbed by the absorbent 15 and such excrement will not leak out of the article 11 while the absorbent article 11 is worn by the user.

The standing portions 16 are a separate member from the sheets 13 and 14, and a side edge of each standing portion 16 is attached to the surface sheet 13 and the anti-leak sheet 14 (see, for example, Japanese Laid-Open Patent Publication Nos. 2001-252308 and 2002-315781).

### SUMMARY OF THE INVENTION

An absorbent article of the present invention is an absorbent article, wherein: a cuff is provided on a surface of a sheet covering a liquid-holding absorbent; the cuff is obtained by folding the sheet, which faces a surface of a body of a wearer, and folding back the sheet so as to form a two-folded shape; and an adhesion section for keeping the two-folded shape of the cuff is provided at least near a base portion of the cuff.

The cuff includes a pair of portions obtained by bending the sheet, which faces the surface of the body of the wearer, and then folding back the sheet. The portions may be bonded to each other at the base portion, or near-base portions of the standing portions may be bonded to another sheet. In one example the cuff has a hairpin-like cross section as the sheet is folded back at the tip portion.

In the present invention, the cuff may be formed from a liquid-permeable surface sheet or a liquid-impermeable anti-leak sheet, or by using both of the surface sheet and the anti-leak sheet. Moreover, in a case where the base portions of the standing portions are bonded to each other in order to keep the shape of the cuff, while the standing portions may be bonded to each other substantially across the entire length of the cuff, it may be sufficient that at least near-base portions of the standing portions are bonded to each other to keep the shape of the cuff.

The standing portions of the cuff may be bonded to each other via a hydrophobic adhesive, or heat seal.

In one embodiment of the present invention, the cuff includes a pair of standing portions facing each other and connected to each other at a folded-back portion; one of the pair of standing portions stands up from, while being connected to, a first surface portion of the sheet; the other one of the pair of standing portions stands up from, while being connected to, a second surface portion of the sheet; and the first surface portion, the pair of standing portions and the second surface portion are formed from a single sheet.

In such a case, the sheet may be liquid-permeable and form a member that covers a part or whole of the absorbent.

Another absorbent article of the present invention at least includes a main part, including a liquid-permeable surface sheet, a liquid-impermeable anti-leak sheet, and a liquid-holding absorbent provided between the surface sheet and the anti-leak sheet, wherein the surface sheet is folded to form standing portions, and the standing portions are bonded together via a hydrophobic first adhesive.

Each standing portion is formed by folding back the surface sheet. The standing portions formed by folding back the liquid-permeable surface sheet are bonded together by a hydrophobic adhesive, whereby excrement such as menstrual blood is prevented from passing through the standing portions.

As a result, it is no longer necessary to use special hydrophobic non-woven fabric for forming the standing portions, whereby the provision of the standing portions does not add to the number of types of materials, and it is possible to reduce the material cost and thus the overall production cost. Moreover, when producing the absorbent article of the present invention, the number of types of materials to be supplied is small, whereby it is possible to reduce the number of pieces of material-supplying equipment.

In the present invention, an elastic member may be bonded, in its stretched state, to the standing portion via a second adhesive, whereby it is possible to stand up the standing portions with respect to the main part when the main part is unfolded from its folded position.

A method for manufacturing an absorbent article of the present invention includes the steps of: forming two cuffs along side edge portions of a web by bending up the side edge portions of the web along lines parallel to a flow direction of the web; and covering an absorbent with the web so that at least a portion of the absorbent is placed between the two cuffs.

Another method for producing an absorbent article of the present invention includes the steps of: placing an elastic member on a web via an adhesive along a flow direction of the web; folding in two a portion of the web where the elastic member is placed so as to sandwich the elastic member therein, and bonding the webs to each other so as to keep the two-folded shape, thereby forming a cuff; and placing an absorbent on the web on which the cuff has been formed.

As described above, according to the present invention, the cuff or the standing portions are formed from the sheet that covers (or wraps) the absorbent, whereby it is no longer necessary to form the cuff or the standing portions from a member separate from the surface sheet and/or the anti-leak sheet and to bond the cuff or the standing portions to the sheets. Thus, it is possible to significantly reduce the production cost.

Moreover, it is possible to maintain the function of the cuff or the standing portions without forming the cuff or the standing portions from a material of a different type from those of the other sheets, whereby it is possible to reduce the material cost. Furthermore, with the present production method, it is possible to easily make the cuff on the surface of the sheet.

### BRIEF DESCRIPTION OF THE DRAWINGS

FIG. **1** is a cross-sectional view illustrating a sanitary napkin, which is an absorbent article according to Embodiment 1 of the present invention.
FIG. **2** is a cross-sectional view illustrating Embodiment 2 of the sanitary napkin.
FIG. **3A** is a cross-sectional view schematically illustrating a sanitary napkin, which is an absorbent article according to Embodiment 3, and FIG. **3B** and FIG. **3C** are schematic cross-sectional views illustrating alternative sanitary napkins.
FIG. **4A** and FIG. **4B** are a plan view and a schematic cross-sectional view, respectively, illustrating a sanitary napkin.
FIG. **5** is a perspective view illustrating an example of a sanitary napkin as a conventional absorbent article.
FIG. **6** is a cross-sectional view of the sanitary napkin of FIG. **5**, taken along line I-I.
FIG. **7** is a schematic diagram illustrating an example of production equipment for producing an absorbent article of the present invention.
FIG. **8A** is a plan view illustrating a web in step 1, and FIG. **8B** is a cross-sectional view taken along line Y-Y of FIG. **8A**.
FIG. **9A** and FIG. **9C** are plan views each illustrating a web in step 2, and FIG. **9B** and FIG. **9D** are cross-sectional views taken along line Y-Y of FIG. **9A** and FIG. **9C**, respectively.
FIG. **10A** is a plan view illustrating a laminate obtained in step 4, FIG. **10B** is a cross-sectional view taken along line Y-Y of FIG. **10A**, FIG. **10C** is a plan view illustrating an absorbent article obtained in step 5, and FIG. **10D** is a cross-sectional view taken along line Y-Y of FIG. **10C**.

### DESCRIPTION OF THE PREFERRED EMBODIMENTS

Embodiments of the present invention will now be described with reference to the drawings.

### EMBODIMENT 1

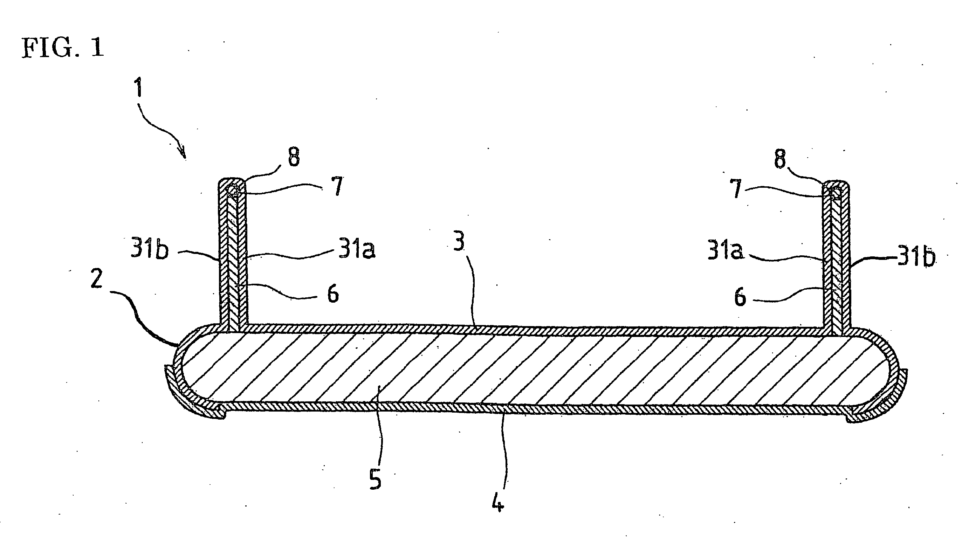

FIG. 1 illustrates Embodiment 1 of a sanitary napkin 1, which is an absorbent article of the present invention. The sanitary napkin **1** includes an elongate main part **2**, including a liquid-permeable surface sheet **3**, a liquid-impermeable anti-leak sheet **4**, and a liquid-holding absorbent **5** provided between the surface sheet **3** and the anti-leak sheet **4**.

The surface sheet **3** is folded to form pair of standing portions **31a** and **31b** along each side edge of the main part **2**. Each pair of standing portions **31a** and **31b**, folded back onto each other, are bonded together via a first adhesive **6**. Moreover, an elastic member **7** is bonded, in its stretched state, to the standing portions **31a** and **31b** via a second adhesive **8** for standing up the standing portions **31a** and **31b** with respect to the main part **2** when the main part **2** is unfolded from its folded position into its ready-to-use position. Note that the elastic member **7** may be a single strand or a plurality of strands.

The height of the standing portion **31a** and **31b** is preferably about 2 to 40 mm so as to prevent excrement from leaking out of the main part **2**. Therefore, at least the first adhesive **6** is hydrophobic, and is applied with a coater or a curtain spray. Note that while the first adhesive **6** and the second adhesive **8** may be of the same material (e.g., a hot melt), the second adhesive **8** is preferably applied on the elastic member **7** with a comb gun, a spiral gun or an omega gun. This is because the appearance of the gathers formed along the standing portions **31a** and **31b** can be improved by applying the second adhesive **8** at separate portions of the elastic member **7**.

Although not specifically depicted in the figure, the anti-leak sheet 4 includes an adhesive application section extending in the longitudinal direction on which an adhesive is applied, with a release liner being attached to the adhesive application section.

### EMBODIMENT 2

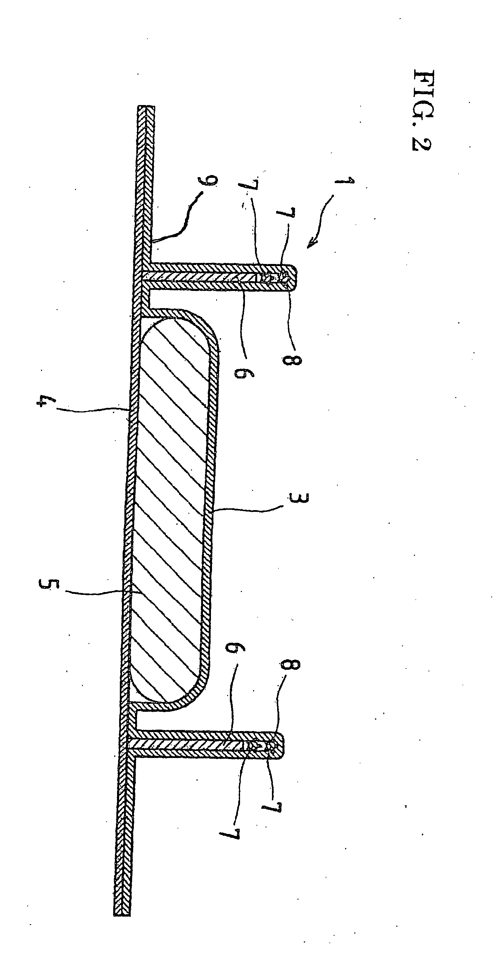

FIG. **2** illustrates Embodiment 2 of the sanitary napkin **1**, which is an absorbent article of the present invention. The sanitary napkin **1** includes a main part **9**, which is substantially the same as the main part **2** illustrated in FIG. **1** except that the standing portions **31** are not located above the absorbent **5**. The surface sheet **3** is connected to the anti-leak sheet **4** around the absorbent **5** viaheat seal or an adhesive. In a case where an adhesive is used, the adhesive may be applied with a spiral gun. The sanitary napkin **1** of FIG. **2** includes a generally trapezoidal wing portion along each side edge of the main part **9**.

Next, how to use the sanitary napkin **1** having such a structure will be described. As the folded sanitary napkin **1** is unfolded into its ready-to-use position, the elastic member **7** is stretched while being urged to its un-stretched position so that the elastic member **7** is stretched over the shortest distance between the opposite ends thereof, whereby the standing portions **31** stand up with respect to the main part **2**. Then, the release liner attached to the anti-leak sheet **4** is peeled off from the adhesive application section, and the adhesive application section is attached to the crotch portion of the inner surface of the user's underwear. Then, the underwear with the sanitary napkin **1** attached thereon is worn by the user, whereby the standing portions **31** come into contact with the user's skin along with the surface sheet **3**.

Then, while excrement is absorbed by the absorbent **5** via the surface sheet **3**, it is prevented from leaking out by the anti-leak sheet **4**. Even if excrement reaches the standing portions **31**, excrement is prevented from passing through the standing portions **31** because they are bonded to each other via the first adhesive **6**.

### EMBODIMENT 3

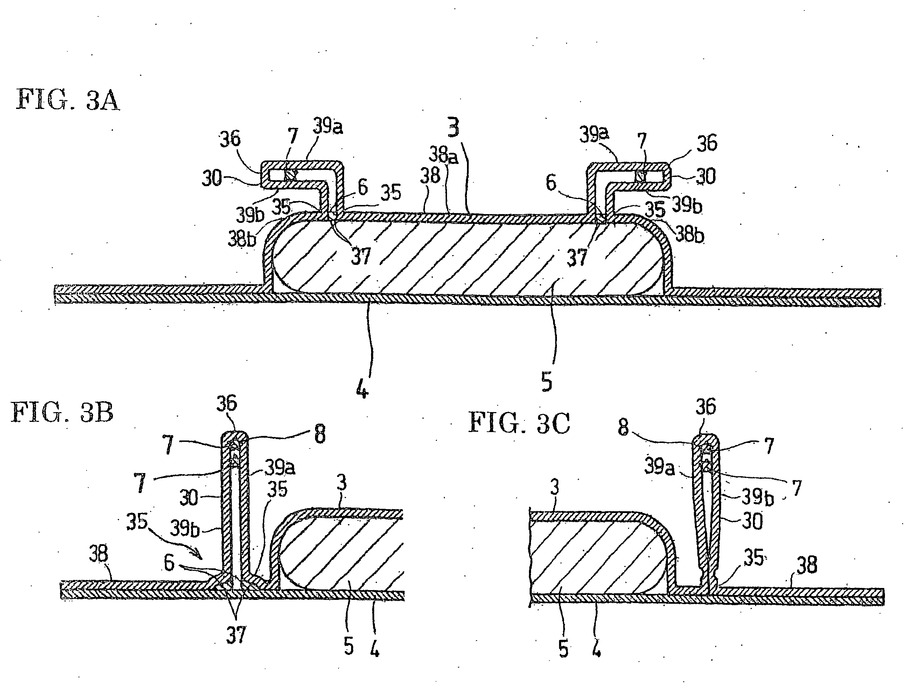

Next, Embodiment 3 of the present invention will be described. FIG. **3A** to FIG. **4B** illustrate Embodiment 3. As illustrated in FIG. **3A**, a pair of anti-leak cuffs **30** are provided on the surface of the surface sheet **3** covering the inner side of the liquid-holding absorbent **5**. Each cuff **30** includes a pair of standing portions **39a** and **39b** obtained by bending the surface sheet **3**, which faces the surface of the body of the wearer, so as to stand up from a surface portion **38** of the surface sheet **3**, and then folding back the surface sheet **3**. Therefore, the cuff **30** has a hairpin-like cross section as the sheet is folded back at a tip portion (folded-back portion) **36**. Note that while FIG. **3A** shows an example where the cuffs **30** are bent outwardly, the cuffs **30** may alternatively be bent inwardly.

Thus, as illustrated in FIG. **3A**, the pair of standing portions **39a** and **39b** are bonded to each other while facing each other and being connected to each other at the tip portion **36**. A first surface portion **38a** of the surface sheet **3** is connected to a base portion **35** of one of the pair of standing portions **39a**, and a second surface portion **38b** of the surface sheet **3** is connected to the base portion **35** of the other standing portion **39b**. The first surface portion **38a**, the pair of standing portions **39a** and **39b**, and the second surface portion **38b** are formed from the single surface sheet **3** with such a structure.

In the present embodiment, the pair of cuffs **30** are provided, with the first surface portion **38a** being located between the pair of cuffs **30**, so that the surface sheet **3** forms a member that covers a portion of the absorbent **5**, as illustrated in FIG. **3A**. The first surface portion **38a** may alternatively form a member that covers the entirety of the absorbent **5**.

An adhesive portion **37** may be provided between the base portions **35** of each pair of standing portions **39a** and **39b**. By applying the first adhesive **6** on the adhesive portion **37**, the base portions **35** of the standing portions **39a** and **39b** are bonded together, thereby keeping each cuff **30** in its two-folded shape. Note that the first adhesive **6** is applied only to the adhesive portion **37**, and is not applied to any other portions between the standing portions **39a** and **39b**.

After excrement reaches the cuff **30**, the excrement is directed by the cuff **30** downwardly (toward the absorbent **5**) so as to be absorbed by the absorbent **5** via the surface sheet **3**. Therefore, even if the cuff **30** is formed from the liquid-permeable surface sheet **3**, it is possible to prevent a leak of excrement to some degree.

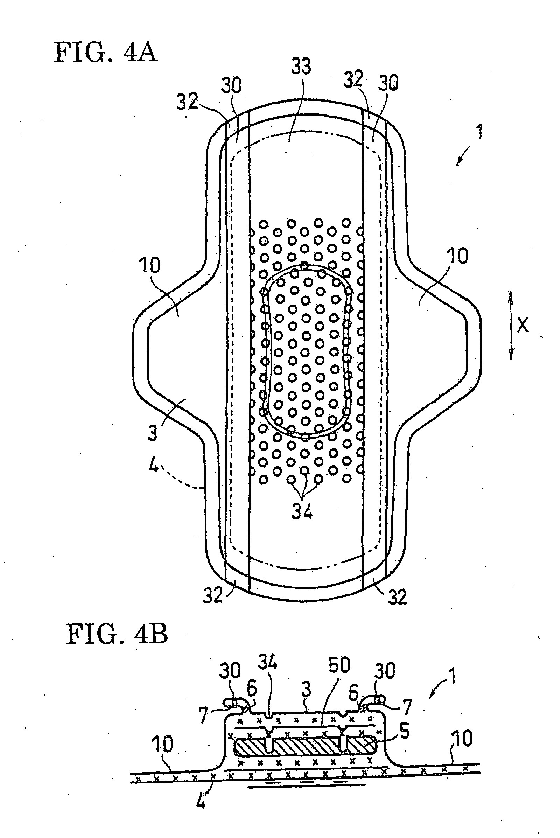

The surface sheet **3** illustrated in FIG. **4A** and the anti-leak sheet **4** on the reverse surface may be attached to each other via heat embossing along the periphery thereof. In such a case, opposite ends **32** of each cuff **30** in the longitudinal direction X may be fixed to the surface sheet **3**, with the cuffs **30** being folded onto the surface of the surface sheet **3**.

On the other hand, referring to FIG. **4B**, the surface sheet **3** and the absorbent **5** may be bonded to each other via heat seal or an adhesive. The surface sheet **3** may be bonded to the absorbent **5** in an adhesion area **33** that is shown by a two-dot chain line and a broken line in FIG. **4A**.

A transfer sheet **50** may be inserted between the surface sheet **3** and the absorbent **5**.

The adhesion area **33** may be embossed. The shape of holes **34** formed by the embossing process may be rectangular or rhomboidal as well as circular as illustrated in FIG. **4A**.

Moreover, as illustrated in FIG. **3B** and FIG. **3C**, the cuff **30** may be provided in a portion where the surface sheet **3** is bonded to the anti-leak sheet **4**. In such a case, the base portion **35** of the cuff **30** and the anti-leak sheet **4** may be bonded to each other via the first adhesive **6**, as illustrated in FIG. **3B**, thereby keeping each cuff **30** in its two-folded shape.

Moreover, the first and second adhesives **6** and **8** may be applied in the longitudinal direction of the napkin either continuously or intermittently. Note that it is not necessary to use the first adhesive **6** to bond the standing portions **39a** and **39b** to each other. For example, the base portions **35** of each pair of standing portions **39a** and **39b** may be bonded to each other by heat seal, as illustrated in FIG. **3C**.

Other than this, the structure and how to use the article of the present embodiment are similar to Embodiment 1, and the components that are same as, or corresponding to, those of Embodiment 1 will be denoted by the same reference numerals and will not be further described below. Note that while the sanitary napkin 1 has been described in the embodiment above, the present invention is not limited to the sanitary napkin **1**. Moreover, depending on circumstances, the main part **2** may include a generally trapezoidal wing portion along each side edge of the main part **2**, as illustrated in FIG. **4A**, or the main part **9** may not include a wing portion.

Moreover, the standing portion **31** or the cuff **30** may be used not only in sanitary napkins but also in disposable diapers and pants (hereinafter they are both referred to simply as "pants"). In a case where the standing portion is used around each leg hole of the pants, it is possible to prevent excrement from leaking from the pants along the surface of the leg of the wearer. Moreover, in a case of pants for male users, if the standing portion is used for the front portion of the pants, it is possible to prevent excrement from leaking from the front portion of the pants.

Moreover, a transfer sheet for guiding excrement generally in one direction (i.e., from the surface sheet **3** toward the absorbent **5**) may be inserted between the surface sheet **3** and the absorbent **5**. The transfer sheet may have a mesh texture.

Moreover, the absorbent **5** may be embossed from above the surface sheet **3**. In this way, it is possible to adjust the shape of the absorbent **5** and to increase the absorptivity of the absorbent **5**.

### EMBODIMENT 4

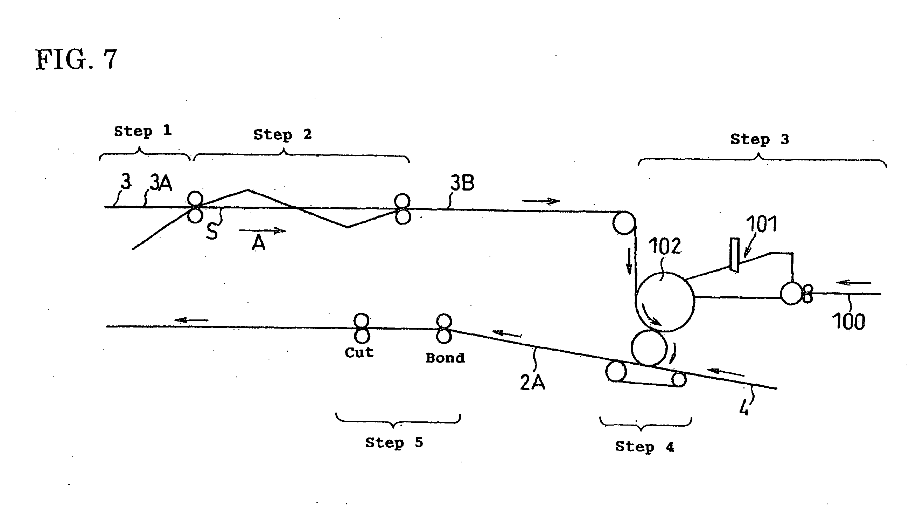

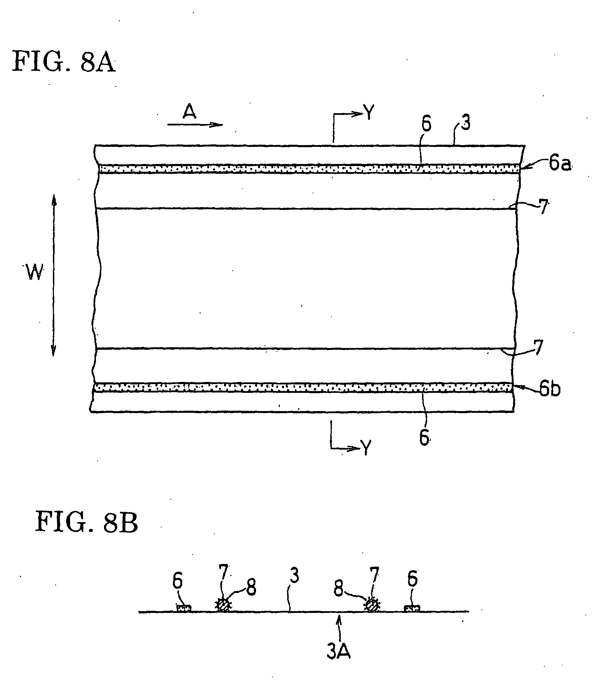

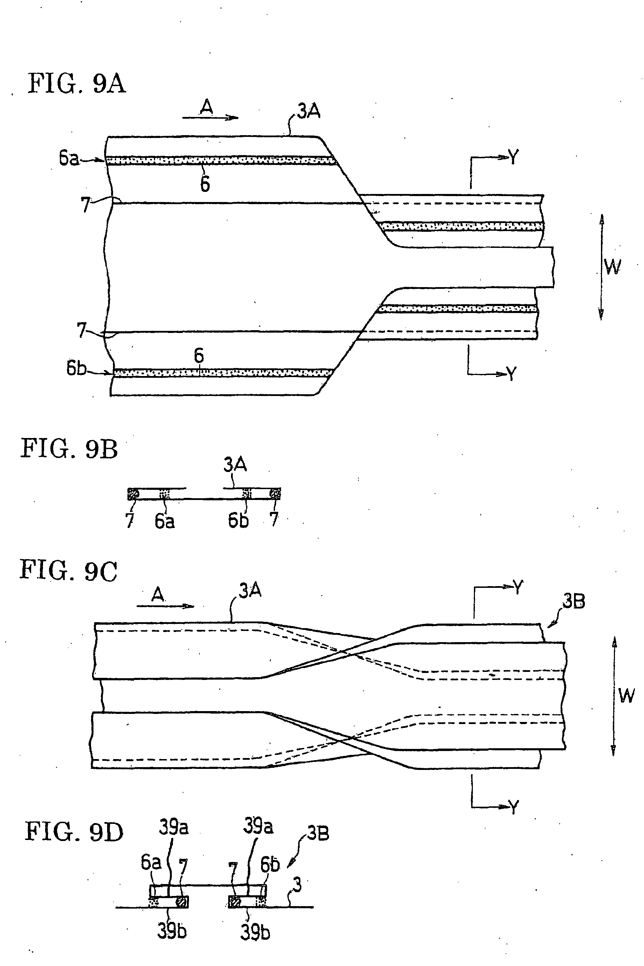

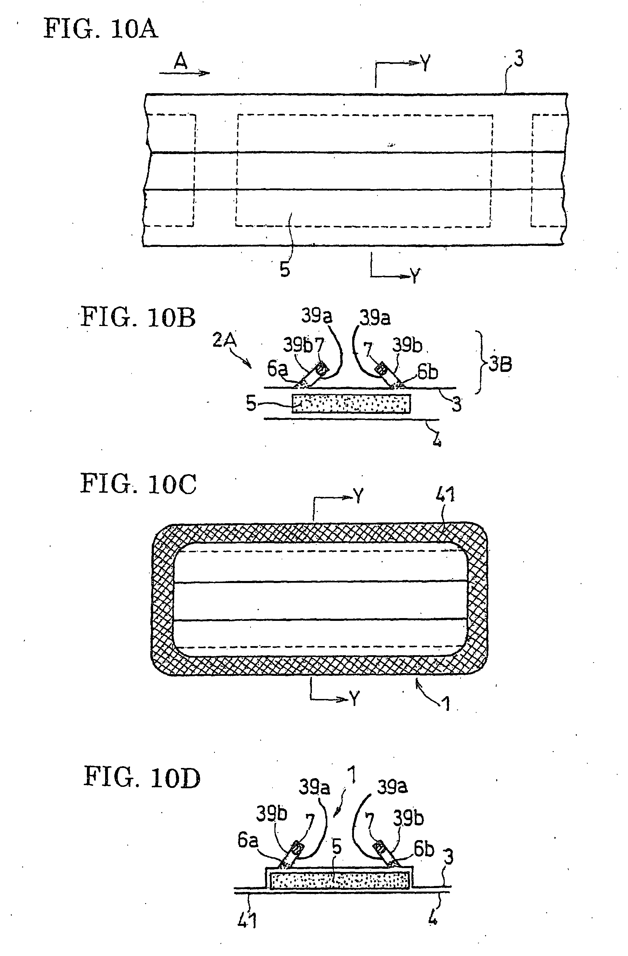

A method for continuously producing absorbent articles of the present invention will be described with reference to FIG. **7** to FIG. **10D**, in which the flow direction of the web corresponds to the longitudinal direction of the absorbent articles. FIG. **7** illustrates production equipment.
Step 1:
   As illustrated in FIG. **8A**, the first adhesives **6** are applied with a coater, a curtain spray, or the like, to edge portions **6a** and **6b**, opposing each other in the width direction W, of non-woven fabric (an example of the web) **3** for the surface sheet that is being fed continuously in the flow direction A. On the other hand, the second adhesives **8** may be applied on the elastic members **7** of FIG. **8B** with a spiral gun, a comb gun, or the like, and the elastic members **7** with the adhesives **8** applied thereon are placed on the non-woven fabric **3**, thereby obtaining a surface sheet **3A** having the elastic member **7** and the adhesion sections **6** and **8**. The first adhesive **6** and the second adhesive **8** may be any water-repelling adhesive (e.g., a hot melt), and the first adhesive **6** and the second adhesive **8** may be the same adhesive. Note that an adhesive that itself is elastic, such as an elastic hot melt, may be used. The widths of the adhesive application sections **6a** and **6b** may be increased. Alternatively, the first adhesive **6** may be applied on a portion of the non-woven fabric **3** where the elastic member **7** is to be placed, in which case the elastic member **7** may be placed on the non-woven fabric **3** without applying the second adhesive **8** to the elastic member **7**. Moreover, the elastic member **7** to be sandwiched between the webs may be a single strand or a plurality of strands.
Step 2:
   The side edges of the surface sheet **3A** obtained in step **1** shown in FIG. **7** are folded inwardly with a sailor **S**, or the like, so that the first adhesive **6** and the elastic member **7** are sandwiched by the surface sheet **3A**, as illustrated in FIG. **9A** and FIG. **9B**. Then, the unbonded portions of the surface sheet **3A** are folded outwardly along the adhesive application sections, as illustrated inFIG. **9C** and FIG. **9D**, thereby obtaining a surface sheet **3B** including the standing portions **39a** and **39b** to be the anti-leak cuffs. Moreover, in a case where hydrophobic non-woven fabric is used as the non-woven fabric **3**, it is preferred that holes are made in the non-woven fabric **3**, thereby making it into a mesh sheet, in order to give some liquid-permeability to the sheet. The step of making holes can be performed at any time before step **3** to be described later.
Step 3:
   On the other hand, referring again to FIG. **7**, a roll pulp **100** is crushed by a crusher **101** to obtain a fibrillated fluff, which is placed on a pattern drum **102** to form the absorbent **5** (FIG. 1). The absorbent **5** may be wrapped by tissue paper, non-woven fabric, or the like. A highly absorbent polymer may be mixed in the fluff. Moreover, the absorbent **5** may be made of an airlaid pulp.
Step 4:
   On the pattern drum **102**, the absorbent **5** is placed onto the surface sheet **3B** having the standing portions **39a** and **39b** obtained in step **2** above, and then the absorbent **5** is placed so that the surface thereof faces the anti-leak sheet **4** to form a laminate **2A** of the surface sheet **3B**, the absorbent **5** and the anti-leak sheet **4**, as illustrated in FIG. **10A** and FIG. **10B**. A transfer sheet may be sandwiched between the surface sheet **3B** and the absorbent **5**. Moreover, before the absorbent **5** is placed so that the surface thereof faces the anti-leak sheet **4**, other non-woven fabric, etc. may be placed between the absorbent **5** and the anti-leak sheet **4**.
Step 5:
   As illustrated in FIG. **10C** and FIG. **10D,** a portion of the laminate **2A** corresponding to a periphery portion **41** of the absorbent article **1** is heat-sealed, and then the laminate **2A** is cut into individual absorbent articles **1**. Before this step, the laminate **2A** may be embossed. Note that while the standing portions **39a** and **39b** are bonded together with an adhesive in the embodiment above, the standing portions **39a** and **39b** may alternatively be bonded by heat seal. Moreover, while a process for producing an absorbent article in which the standing portions **39a** and **39b** are located above the absorbent **5** has been described in the embodiment above, the standing portions **39a** and **39b** may be formed outside the absorbent **5,** as illustrated in FIG. **2,** FIG. **3B** and FIG. **3C**. In such a case, the base portions **35** may be obtained by bonding the surface sheet and the anti-leak sheet with an adhesive or heat seal (see FIG. **3B**). Moreover, the absorbent article **1** may be provided with wing portions **10**, as illustrated in FIG. **4A.** The absorbent article produced in this way may be used not only for sanitary napkins, but also for other types of articles, such as disposable diapers, absorbent pads, etc.
   The present invention may be used for an absorbent article that is put on the inside of the user' s underwear in a narrow crotch area, such as a sanitary napkin, an incontinence pad, a sanitary pad for vaginal discharges, a disposable diaper, or the like.

## Claims

1. An absorbent article (1), comprising:
a cuff (30) formed on a surface of a sheet (3) covering a liquid-holding absorbent (5), wherein the cuff (30) comprises a folding of the sheet (3) to form a two-folded shape, the cuff (30) facing a surface of a body of a wearer; and
an adhesion section (6a, 6b, 37) for maintaining the two-folded shape of the cuff (30), the adhesion section (6a, 6b, 37) provided at least near a base portion (35) of the cuff (30).

2. The absorbent article (1) according to claim 1, wherein:
the cuff (30) includes a pair of portions facing each other and a folded-back portion (36); and
the pair of portions and the folded-back portion (36) are formed from a part of the sheet (3).

3. The absorbent article (1) according to claim 2, wherein the sheet (3) is liquid-permeable and forms a member that covers a part or whole of the absorbent (5).

4. A method for producing an absorbent article (1), comprising:
applying an adhesive (8) on an elastic member (7);
placing the elastic member (7) on a web (3) along a flow direction (A) of the web;
folding the web (3) in two so as to sandwich the elastic member (7) therein, thereby defining a bonded portion and an unbonded portion; and
folding outwardly the unbonded portion of the web (3) along the elastic member (7), thereby defining a two-folded shape of the web.

5. The method for producing an absorbent article (1) according to claim 4, wherein in the step of folding the web (3), at least two cuffs (30) extended in the flow direction (A) are formed, and
an absorbent (5) is covered with the web (3) so that at least a portion of the absorbent (5) is placed between the two cuffs (30).

6. The method for producing an absorbent article (1) according to claim 4, wherein the two-folded shape is further defined by sealing an opening of the two-folded shape of the web (3).

7. The method for producing an absorbent article (1) according to claim 4, wherein the two-folded shape is further defined by bonding an opening of the two-folded shape of the web (3) with an adhesive (6).

8. The method for producing an absorbent article (1) according to claim 4, wherein the step of folding the web (3) in two is performed with a sailor (S).

9. An absorbent article (1), comprising:
a liquid permeable surface sheet (3);
a liquid impermeable anti-leak sheet (4); and
a liquid holding absorbent (5) disposed between the liquid permeable surface sheet (3) and the liquid impermeable anti-leak sheet (4),
wherein portions of the liquid permeable surface sheet (3) are folded to form two standing portions (31a, 31b, 39a, 39b) , on opposing sides thereof and extending along a longitudinal direction (X) of the article, wherein the two standing portions (31a, 31b, 39a, 39b) operate to minimize waste leaking from the article in a direction generally transverse to the longitudinal direction (X) corresponding to the sides of the article.

10. The article of claim 9, wherein the liquid holding absorbent (5) is disposed in and extending longitudinally within a center portion of the article between the liquid permeable surface sheet (3) and the liquid impermeable anti-leak sheet (4), and wherein the two standing portions (31a, 31b, 39a, 39b) reside outside the center portion.

11. The article of claim 9, further comprising a first adhesive disposed within the two folded standing portions (31a, 31b, 39a, 39b) of the liquid permeable surface sheet (3).

12. The article of claim 11, wherein the first adhesive is generally hydrophobic.

13. The article of claim 9, further comprising an elastic member (7) extending longitudinally within each of the standing portions (31a, 31b, 39a, 39b).

14. The article of claim 13, wherein each elastic member (7) resides near a tip portion (36) of a respective standing portion .

15. The article of claim 14, wherein the elastic members reside in the standing portions (31a, 31b, 39a, 39b) in a stretched state, respectively, when the absorbent article (1) is unfolded longitudinally in a ready-to-use position, thereby biasing at least a portion of the standing portions (31a, 31b, 39a, 39b) to extend from a surface of the liquid permeable surface sheet (3) at a base portion (35) in a generally perpendicular direction.

16. The article of claim 9, wherein each standing portion (31a, 31b, 39a, 39b) extends from a surface of the liquid permeable surface sheet (3) at a base portion (35) in a generally perpendicular manner, and wherein each standing portion (31a, 31b, 39a, 39b) has a bent portion associated therewith that extends inwardly toward a center portion of the article or outwardly away from the center portion, the standing portions (31a, 31b, 39a, 39b) forming anti-leak cuffs (30).

17. The article of claim 16, further comprising a first adhesive at the base portion (35) of each standing portion (31a, 31b, 39a, 39b).

18. The article of claim 16, further comprising an elastic member extending longitudinally within the bent portion of each standing portion.

19. The article of claim 16, wherein the liquid holding absorbent (5) is disposed in a center portion between the liquid permeable surface sheet (3) and the liquid impermeable anti-leak sheet (4), and wherein the two standing portions (31a, 31b, 39a, 39b) reside outside the center portion.
